(19) Europäisches Patentamt
European Patent Office
Office européen des brevets

(11) **EP 1 870 035 B1**

(12) **EUROPEAN PATENT SPECIFICATION**

(45) Date of publication and mention
of the grant of the patent:
**30.12.2015 Bulletin 2015/53**

(51) Int Cl.:
***A61B 5/0205*** *(2006.01)*    ***A61B 5/029*** *(2006.01)*
***G06F 19/00*** *(2011.01)*    *A61M 16/00* *(2006.01)*

(21) Application number: **07012002.7**

(22) Date of filing: **19.06.2007**

(54) **Apparatus and computer program for determining a patient's volemic status represented by cardiopulmonary blood volume**

Vorrichtung und Computerprogramm zur Bestimmung des volemischen Status eines Patienten durch dessen Darstellung über das kardiopulmonale Blutvolumen

Appareil et programme informatique pour déterminer un statut volémique d'un patient représenté par le volume sanguin cardiopulmonaire

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **21.06.2006 DE 102006028533**

(43) Date of publication of application:
**26.12.2007 Bulletin 2007/52**

(73) Proprietor: **Edwards Lifesciences IPRM AG**
**1260 Nyon (CH)**

(72) Inventors:
• **Pfeiffer, Ulrich**
**81667 Munich (DE)**

• **Michard, Frédéric**
**91570 Bièvres (FR)**
• **Knoll, Reinhold**
**81543 Munich (DE)**

(74) Representative: **Eisenführ Speiser**
**Patentanwälte Rechtsanwälte PartGmbB**
**Postfach 31 02 60**
**80102 München (DE)**

(56) References cited:
**EP-A- 1 155 658**    **EP-A1- 1 588 661**
**US-A- 5 769 082**    **US-A1- 2003 060 722**
**US-A1- 2003 167 010**    **US-A1- 2004 249 297**
**US-B1- 6 322 514**

**Description**

Field of the invention

The invention relates to an apparatus and a computer program for determining a patient's volemic status represented by cardiopulmonary blood volume CPBV mainly used in fluid management.

Background of the Invention

It is generally known that in the critical-care diagnosis and treatment of critically ill patients, thoracic blood volumes, i.e. intrathoracic blood volume, right and left heart end-diastolic volumes, are important characteristics for monitoring the patient's state of health and for fluid management of such a patient.

According to prior art the thoracic blood volumes can be determined by using a dilution measurement. A bolus of an indicator defined by a predetermined quantity of the indicator is rapidly injected central-venously into the patient's superior vena cava, and the indicator concentration response is measured at a downstream location of the patient's systemic circulation downstream after cardiopulmonary passage in the arterial system as close as possible to the outflow of the left ventricle. Based on the indicator concentration response measurement versus time the dilution curve is generated.

During at least one cardiopulmonary circulation, the indicator mainly remains in the intravascular space. E.g. indocyanine green, Evan's blue, or hypertonic saline indicator can be used as intravascular indicator.

The cardiopulmonary blood volume CPBV is represented by the volume of distribution during cardiopulmonary passage which is defined by the multiplication of the cardiac output CO and the mean transit time TT of the respective intravascular indicator, i.e.

$$CPBV = CO * TT.$$

It is common to determine the cardiac output CO and the mean transit time TT from the dilution curve. Also, it is known to obtain the cardiac output from any method, which simultaneously displays the cardiac output CO, e.g. echocardiography, transthoracic electrical bioimpedance, or continuous heating right heart catheter, or CO2-rebreathing.

The cardiopulmonary blood volume CPBV which is calculated with above equation consists of the largest accessible distribution volumes for the indicator, which are the sum of the end-diastolic volumes of the right atrium, the right ventricle, the maximum of the pulmonary blood volume during several ventilation cycles within the measurement period, the left atrial and the left ventricular end-diastolic volume, and a smaller portion of aortic blood volume, which is more or less constant.

Another known method for determining the cardiopulmonary blood volume CPBV is a variant of the above method, wherein a single indicator is used. By using this single indicator transpulmonary thermodilution technique, only the sum of the atrial and ventricular end-diastolic blood volumes (i.e. global end-diastolic volume) is exactly measured, whereas the pulmonary blood volume is estimated by assuming that it behaves more or less proportional to the global end-diastolic volume.

When using the known indicator dilution techniques for determining the cardiopulmonary blood volume CPBV, the determination can be done not continuously, but discontinuously, not automatically, but the determination requires user interaction and is labour and cost intensive.

US patent 5,769,082 discloses an apparatus and a method of assessing cardiovascular function of patients who are mechanically ventilated, in particular a method of ventilation. During ventilation, the reactiveness of the patient to a predetermined sequence of ventilation cycles of varying volumes is used for such assessments. The respiratory maneuver is composed of e.g. four consecutive ventilation steps using volumes of varying magnitudes. The volumes and the time differences between the ventilation steps are calculated on the basis of the patients. Blood pressure curves may be obtained in response to said respiratory maneuver, as an example of a hemodynamic parameter.

In US patent application 2004/0249297, an apparatus is disclosed for determining cardiovascular parameters characterising a patient's left ventricular pumping action without the lessening of the validity of measuring results through the effect of respiration and changing respiratory states. Timing sequences of the thoracic dimension and central venous pressure may be obtained. By means of conventional algorithms of pulse counter analysis the apparatus calculates a left ventricular stroke volume and a stroke volume variation or additionally other desired cardiovascular readings. A pulse shift between the beginning of the rise of the thoracic dimension curve and the beginning of the rise of curve of the central venous pressure indicates whether mechanical respiration or spontaneous breathing is taking place.

In European patent application 1 155 658, a system and a method is disclosed for automatic evaluation of the index of volumetric status (systolic pressure variation SPV) of a patient. The measuring procedure includes two periods, one period of mechanical breathing and another period of apnoea which preferably follows the period of mechanical. Values of systolic peaks of every heart cycle in the two periods are determined. Indexes of volumetric status are calculated. To determine values of systolic peaks, the main frequency of the signal and the spectrum of frequency is determined by means of a Fast Fourier Analysis.

In US patent 6,322,514, a method for determining cardiac characteristics of subject is disclosed. To determine cardiac characteristics, such as stroke volume, cardiac output, ejection time or the like, the method makes use of the fact, that the thoracic cavity and the abdominal cavity contain a number of hollow organs, as heart, lungs, the digestive system, bladder or kidneys. Volumetric changes occurring while respiration or beating of the heart lead to a change in pressure in said hollow organs. The incremental quantity of breathing gas is measured and relates to the quantity of change in pressure in thoracic cavity which is also measured by a respective catheter containing a pressure measure device, like a pressure transductor.

It is an object of the invention to provide an apparatus and a computer program for determining a patient's volemic status represented by cardiopulmonary blood volume CPBV, wherein the determination is continuous and easy to achieve.

Summary of the invention

According to the invention, this object is achieved by an apparatus for determining a patient's volemic status, adapted to make use of a physiological heart-lung interaction during spontaneous breathing or mechanical ventilation as defined in claim 1. Further, this object is achieved by a computer program for determining a patient's volemic status, as defined in claim 14.

Due to the fact that using the physiological heart-lung interaction during spontaneous breathing or mechanical ventilation for determining the cardiopulmonary blood volume CPBV, the determination can be done continuously and automatically without the user's interaction. Therefore, the inventive apparatus and the inventive computer program perform a less labour intensive and less cost intensive determination of a patient's volemic status.

The apparatus is adapted to provide an envelope of the arterial pulse pressure and is capable to determine the physiological heart-lung interaction during mechanical ventilation or spontaneous breathing by making use of the envelope of the arterial pulse pressure.

Furthermore, the computer program has instructions adapted to carry out the steps of providing an envelope of the arterial pulse pressure, and determining the physiological heart-lung interaction by making use of the envelope of the arterial pulse pressure.

The apparatus is capable to derive the expiratory cardiopulmonary blood volume CPBVex by making use of the equation

$$CPBVex = CO * TTcp,ex,$$

wherein CO is the cardiac output and TTcp,ex is the cardiopulmonary transit time of blood in the hemodynamic status of expiration being derived from the envelope of the arterial pulse pressure. Also, the computer program has instructions adapted to carry out the step of deriving the expiratory cardiopulmonary blood volume CPBVex by making use of above equation.

It is preferred that the apparatus is capable to derive the inspiratory left heart volume LHVin by making use of the equation

$$LHVin = CO * TTlh,in,$$

wherein CO is the cardiac output and TTlh,in is the inspiratory transit time of blood through the left heart being derived from the envelope of the arterial pulse pressure. Also, the computer program is preferred to have instructions adapted to carry out the step of deriving the inspiratory left heart volume LHVin by making use of above equation.

Alternatively, it is preferred that the apparatus and the instructions of the computer program are adapted to be capable to derive a middle expiratory cardiopulmonary blood volume CPBV by making use of the equation

$$CPBV = CO * TTcp,$$

wherein CO is the cardiac output and TTcp is middle cardiopulmonary transit time ranging between the cardiopulmonary transit time of blood TTcp,ex in the hemodynamic status of expiration, and the inspiratory transit time TTlh,in of blood through the left heart, both being derived from the envelope of the arterial pulse pressure.

The apparatus and the computer program are capable to derive the cardiopulmonary transit time of blood in the hemodynamic status of expiration TTcp,ex by making use of the equation

$$TTcp,ex = t(B) - t(I-E),$$

wherein t(I-E) is the time point of end-inspiration and start of expiration, and t(B) is the time point where the envelope of arterial pressure reaches the same level as at the time point of end-expiration and start of inspiration.

Preferably the apparatus and the computer program are capable to derive the inspiratory transit time TTlh,in by making use of the equation

$$TTlh,in = t(E-I) - t(A),$$

wherein t(E-I) is the time point of end-expiration and start of inspiration, and t(A) is the time point where the envelope of arterial pressure starts to rise.

Preferably the apparatus is capable to obtain the cardiac output CO from a continuous real time cardiac output measurement method like e.g. arterial pulse contour analysis, esophageal Doppler, transthoracic or esophageal echo Doppler, transthoracic or esophageal electrical Bioimpedance. It is also preferred that the computer program has instructions being adapted to carry out the step of obtaining the cardiac output from above method.

Further, preferably the apparatus is capable to initially check the equilibrium in the cardiopulmonary vascular system by a single extended breathing cycle in investigating as to whether a constant plateau of pulse pressure for expiration is reached in order to necessarily adjust the breathing cycle to a degree with approximate equilibrium. Also it is preferred that the computer program has instructions adapted to carry out the step of above initially checking.

Preferably the apparatus is capable and preferably the computer program has instructions adapted to apply the checking of equilibrium in a pressure controlled ventilation mode or in a volume controlled ventilation mode.

Alternatively, it is preferred that the apparatus is capable to use prolonged step changes of the level of Positive End-Expiratory Pressure (PEEP), and alternatively, it is preferred that the computer program has instructions adapted to carry out the step of using prolonged step changes of the level of Positive End-Expiratory Pressure (PEEP).

As a further alternative, it is preferred that the apparatus is capable to use prolonged step changes of the level of Positive End-Expiratory Pressure PEEP by breathing on three different mean airway pressure (MPaw) levels, and alternatively, it is preferred that the computer program has instructions adapted to carry out the step of using prolonged step changes of the level of Positive End-Expiratory Pressure PEEP by breathing on three different mean airway pressure (MPaw) levels.

Preferably, the apparatus is adapted to compose a phase of low PEEP level PEEP 1, a phase of high PEEP level PEEP 3, and a phase of intermediate PEEP level PEEP 2. Preferably the computer program has instructions adapted to carry out such composing.

It is preferred that the apparatus is adapted to compose the phase of intermediate PEEP level PEEP 2 corresponding to the mean airway pressure Paw mean before a testing phase PEEP 2. Preferably the computer program has instructions adapted to carry out such composing.

Preferably the apparatus is capable to derive the mean cardiopulmonary blood volume CPBVmean by making use of the equation

$$CPBVmean = COmean * TTcp\ mean,$$

wherein COmean is the mean cardiac output CO in the mean positive airway pressure phase after stabilization and TTcp mean is the mean cardiopulmonary transit time of blood being derived from the envelope of the arterial pulse pressure. It is also preferred that the computer program has instructions adapted to carry out the step of deriving the mean cardiopulmonary blood volume CPBVmean by making use of such equation.

Preferably the apparatus is capable to derive the mean cardiopulmonary transit time of blood TTcp mean by making use of the equations

$$\text{TTcp mean} = t(D) - t(3-2),$$

or

$$\text{TTcp mean} = t(F) - t(1-2),$$

wherein t(3-2) is the moment of change from the highest level of PEEP or MPaw PEEP 3 to mean positive airway pressure level or the intermediate level of PEEP or MPaw PEEP 2, t(1-2) is the moment of change from the lowest level of PEEP or MPaw PEEP 1 to average positive airway pressure level or the intermediate level of PEEP or MPaw PEEP 2, t(D) is the time point where the envelope of arterial pulse pressure curve has adapted to low PEEP level PEEP 1 or MPaw level, and t(F) is the time point where the envelope of arterial pulse pressure curve has adapted to PEEP or MPaw intermediate level PEEP 2.

The apparatus is preferred to be capable and the computer program has preferred instructions being adapted to derive the mean left heart volume LHVmean by making use of the equation

$$\text{LHVmean} = \text{COmean} * \text{TTlh,mean},$$

wherein COmean is the mean cardiac output CO in the mean positive airway pressure phase after stabilization and TTlh,mean is the mean transit time of blood being derived from the envelope of the arterial pulse pressure. The computer program preferably has instructions adapted to carry out the step of deriving the mean left heart volume LHVmean by making use of such equation.

Further, preferably the apparatus is capable to derive the mean transit time of blood TTlh,mean by making use of the equation

$$\text{TTlh,mean} = t(1-2) - t(E)$$

wherein t(E) is the time point where the envelope curve of arterial pulse pressure starts to rise. Preferably the computer program has instructions being adapted to carry out such determination.

Preferably the apparatus is capable and preferably the computer program has instructions adapted to derive the slope of Starling curve during the triphasic positive airway pressure (TriPAP) ventilation/respiration mode for the total heart by making use of the difference quotient ? built from delta SV over delta CPBV on 3 PEEP or MPaw levels, and calculating the slope of the curve fitted through these 3 points,
and similarly for the left heart by making use of the difference quotient built from
delta SV over delta LHV on 3 PEEP or MPaw levels.

Brief Description of the Drawings

In the following the invention is explained on the basis of preferred embodiments with reference to the drawings. In the drawings:

Figure 1 is a first diagram showing a curve of the airway pressure, a bar graph of the arterial pressure, and an envelope of the pulse pressure,

Figure 2 is a second diagram showing a curve of the airway pressure, a bar graph of the arterial pressure, and an envelope of the pulse pressure, and

Figure 3 is a third diagram showing a curve of the airway pressure, a bar graph of the arterial pressure, and an envelope of the pulse pressure.

Detailed Description of preferred Embodiments of the Invention

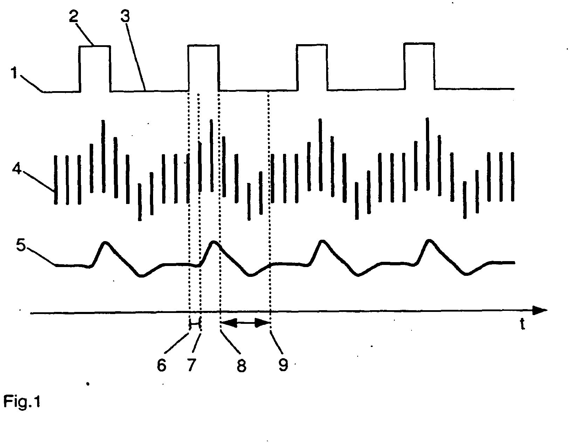

An inventive apparatus is adapted to provide a diagram shown in figure 1. In the diagram a curve of the airway pressure 1 comprising successive inspiration phases 2 and expiration phases 3 is shown, wherein during the inspiration phases 2 the airway pressure is higher than during the expiration phases 3. Further, in the diagram an arterial pressure diagram 4 is shown, wherein for each heart beat a vertical bar reaching from diastolic pressure (minimum pressure) up to systolic pressure (maximum pressure) is plotted. Additionally, in the diagram an envelope of the arterial pulse pressure 5 is shown, which is defined as being the difference between the systolic pressure and the diastolic pressure according to the arterial pressure diagram 4.

The mammalian thorax can be regarded as a chamber with a variable volume. The chamber is composed of the partial volumes of the heart, the lungs, the large extra cardiac vessels, connective tissue and the esophagus. The thoracic volume changes regularly with breathing or mechanical ventilation. Under pathophysiological conditions, it may vary due to increased abdominal pressure, and also due to external pressure, for example during diving, etc..

Looking at a variable thoracic volume in terms of time, it contains partial volumes which change very rapidly, for example within seconds, in the course of a breathing or ventilation cycle, as does the gas volume inside the lungs and the blood volume inside large vessels and inside the heart, and partial volumes which change over longer time periods, such as the functional residual volume of the lungs due to therapeutic intervention, e.g. application of positive end-expiratory pressure, an increase in extravascular lung water (e.g., when pulmonary edema is formed), and an increase in pathological partial volumes (e.g., as in case of hematothorax, pneumothorax or pleural effusion).

In the case of spontaneous breathing, the inhaled air enters the lungs due to the negative intrathoracic pressure ITP which is produced by the thoracic intercostal musculature and the diaphragm. However, the venous blood flow into the chest region, often called venous return, is facilitated during spontaneous inhalation as well. During exhalation in spontaneous breathing, the intrathoracic pressure becomes positive again, which causes gas to leave the lungs, since the pressure within the lungs exceeds atmospheric pressure, while the venous return is slowed down. Exactly the same happens during mechanical respiration when spontaneous breathing is simulated by means of a chamber respirator in the form of an iron lung.

During mechanical respiration, in particular positive-pressure ventilation, inhaling is accomplished by producing a positive gas pressure in the airways by the mechanical ventilator outside the lungs. Respiratory gas enters the lungs because the airway pressure inside the lungs is lower. Gas enters the lungs until the airway pressure in the external airways and the airway pressure in the lungs and internal airways reach equilibrium. During this inhalation process, the lungs are inflated, which increases the intrathoracic pressure, and the large blood vessels (intrathoracic vena cava and aorta) and the heart itself are compressed.

From the physiological point of view, this means that venous blood flow to the right heart, i.e. venous return, is reduced. Exhalation occurs due to the retractive force of the thoracic walls, the diaphragm, and the lungs themselves and, to a lower degree, due to the weight of the thoracic wall itself, whereby the intrathoracic pressure ITP drops again while the venous return increases.

The apparatus is adapted to use interactions between the heart and the lungs during breathing and in particular during mechanical ventilation. Therefore, the above described changes in venous return during spontaneous breathing as well as during mechanical positive pressure ventilation do have a direct effect on the cardiac filling and - via the Frank Starling mechanism - on the ventricular output, i.e. the cardiac stroke volume. The Starling mechanism describes a relationship between the diastolic cardiac filling volume and the systolic cardiac stroke volume to that effect that the more a cardiac chamber is filled in the diastolic phase, the greater is the output of systolic cardiac stroke volume.

In timely manner the following occurs in the cardio-pulmonary vascular system during mechanical positive pressure ventilation with inflation and deflation of the lungs:

- With beginning of inflation of the lungs venous return and right ventricular filling and stroke output decrease, pulmonary blood volume, i.e. blood in the lungs, is squeezed out of the lungs and for a short period of time increases left ventricular filling and stroke volume output.

- When the blood in the lungs is completely squeezed out (at end of inflation) also left ventricular filling and consequently left ventricular stroke volume output decrease.

- With beginning of deflation (i.e. expiration) venous return to the right ventricle and right ventricular stroke volume output start to increase again.

- When the blood volume in the lungs has come up to its new equilibrium also left ventricular filling and stroke volume output have increased to their level right before the start of the mechanical breath.

When the ventilation rate, each of the durations of the inspiration phases 2, and each of the durations of the expiration phases 3 are long enough, equilibrium in the cardiopulmonary vascular system can occur in each of the inspiration phases and the expiration phases.

Referring to figure 1, the apparatus is capable to calculate the cardiopulmonary transit time of blood TTcp,ex

in the hemodynamic status of expiration from the course of the arterial pulse pressure wave envelope 5 by using the equation

$$TTcp,ex = t(B) - t(I-E),$$

wherein t(I-E) is defined as being the time point 8 of end-inspiration and t(B) is defined as being the time point 9 where the envelope of arterial pulse pressure 5 reaches the same level as at as at the time point 6 of end-expiration and start of inspiration.

Under the same conditions and in a similar way the apparatus is adapted to calculate the inspiratory transit time TTlh,in of blood through the left heart (i.e. left atrium and ventricle) by using the equation

$$TTlh,in = t(E-I) — t(A),$$

wherein t(E-I) is the time point 6 of end-expiration and t(A) is the time point 7 where the envelope curve of arterial pulse pressure 5 starts to rise.

Further, the apparatus is adapted to multiply the respective transit time by the respective mean cardiac output CO during the respective time period in which the respective transit time TT has been determined. Therefore, the apparatus is adapted to calculate the expiratory cardiopulmonary blood volume CPBVex and the inspiratory left heart blood volume LHVin using the equations

$$CPBVex = CO * TTcp,ex,$$

and

$$LHVin = CO * TTlh,in,$$

respectively.

The apparatus is adapted to obtain the cardiac output CO from any continuous real time cardiac output measurement method like arterial pulse contour analysis, esophageal Doppler, transthoracic or esophageal echo Doppler, transthoracic or esophageal electrical Bioimpedance or others.

Preferably the apparatus is adapted to initially check the equilibrium in the cardiopulmonary vascular system by a single extended breathing cycle. Thereby a constant plateau of pulse pressure for expiration must be reached. This principle could be applied in a pressure controlled ventilation mode (as shown in figure 1), or in a volume controlled ventilation mode (not shown in figure 1). Afterwards the breathing cycle is adjusted to a degree where equilibrium is approximately reached.

Alternatively, the apparatus is adapted to calculate the cross correlation between airway pressure and pulse pressure even in spontaneous breathing patients with irregular breathing patterns. The delay of the maximum peak of the cross correlation function would provide a middle cardiopulmonary transit time TTcp which ranges in between TTcp,ex and TTlh,in. The middle transit time TTcp multiplied with the mean cardiac output CO is to be used to estimate a middle cardiopulmonary blood volume, i.e.

$$CPBV = CO * TTcp.$$

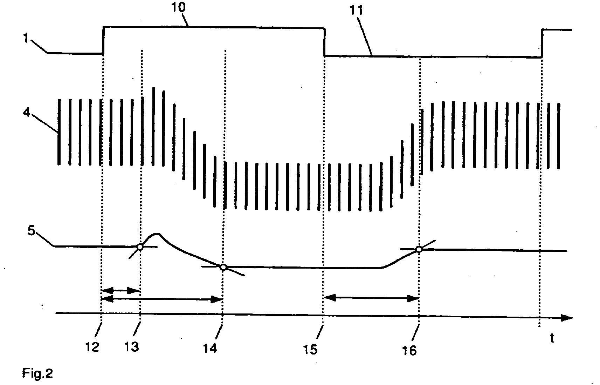

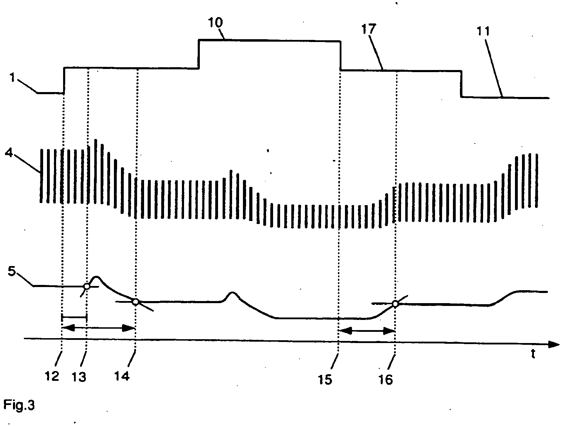

The apparatus is further improved by being adapted to use prolonged step changes of the level of Positive End-Expiratory Pressure PEEP or by breathing on three different mean airway pressure levels MPaw, instead of short term phasic changes in airway pressure during a single mechanical breath according to the diagram shown in figure 1. Therefore, the apparatus is adapted to provide diagrams as shown in figures 2 and 3.

In the diagram shown in figure 2 a curve of the airway pressure 1 comprising the successive high PEEP or MPaw levels (PEEP 3) 10 and the low PEEP or MPaw levels (PEEP 1) 11 is shown. Further, in the diagram shown in figure 3 a curve of the airway pressure 1 comprising the successive high PEEP or MPaw levels (PEEP 3) 10, middle PEEP or MPaw levels (PEEP 2) 17 and the low PEEP or MPaw levels (PEEP 1) 11 is shown.

Furthermore, in the diagrams shown in figures 2 and 3, respectively, an arterial pressure diagram 4 is shown, wherein for each heart beat a vertical bar reaching from diastolic pressure (minimum pressure) up to systolic pressure (maximum pressure) is plotted. Additionally, in the diagrams, respectively, an envelope of the arterial pulse pressure 5 is shown, which is defined as being the difference between the systolic pressure and the diastolic pressure according to the arterial pressure diagram 4.

Any change in the PEEP level or MPaw level causes a simultaneous corresponding change of intrathoracic pressure which compresses the low pressure capacitance blood vessel system in the chest and changes venous return, right ventricular and left ventricular preload, and hence stroke volume output. This results in the PEEP / MPaw level analogous phase shifted swings in the envelope of the arterial pulse pressure wave 5.

The PEEP or MPaw procedure is composed of a manoeuver similar to bi-phasic PEEP or MPaw which originally has been introduced as BIPAP (Benzer & Baum 1989); i.e. a phase of low PEEP 11 (PEEP 1) and a phase of high PEEP 10 (PEEP 3), and a third phase on the PEEP level 17 which corresponds to the mean airway pressure (Paw mean) before the testing phase (PEEP 2) (see figure 3). Hence a tri-phasic mean airway pressure pattern results which will be named "TriPAP".

When producing the TriPAP ventilation pattern, it needs to be set on a ventilator either manually or by remote control through the hemodynamic monitor, and the information on the pattern, timing and PEEP or MPaw levels can either be fed into the hemodynamic monitor, which does all calculations, directly via cable or via wireless connection.

Alternatively the information can be obtained from feeding airway pressure or a surrogate for intrathoracic pressure like esophageal pressure or an electrical bioimpedance respiration signal directly into the hemodynamic monitor. Further, any step change in PEEP or MPaw level is also simultaneously and immediately reflected in a corresponding change of central venous pressure, hence this information can also be obtained from direct analysis of central venous pressure in the hemodynamic monitor itself.

The apparatus is adapted to use the TriPAP mode as normal either controlled ventilation mode or during spontaneous ventilation as well with the later being superimposed. Once this ventilation TriPAP is set as continuous ventilation/respiration mode, the hemodynamic test can be automatically repeated continuously as well.

The measurement of the respective transit time for calculation of cardiopulmonary blood volume CPBV and the inspiratory left heart volume LHEV is of clinical interest mainly during Paw mean conditions. The respective PEEP or MPaw levels are kept until stabilization of the arterial pulse pressure curve envelope occurs, which is observed when the cardiopulmonary blood volume CPBV has adapted to the new PEEP or MPaw level, hence some seconds later than the cardiopulmonary transit time TTcp.

The apparatus is adapted to calculate the cardiopulmonary transit time TTcp in the phases starting with switching to mean airway pressure coming either down from the highest level 10 of PEEP or MPaw (at time 15 t(3-2)) or coming up from the lowest level 11 of PEEP or MPaw (at time 12 t(1-2)) in two ways :

- from the beginning of the step change of PEEP or MPaw until the crossing point of the line of backward extrapolation of the pulse pressure envelope with the tangent at the steepest point on the downslope or upslope of the pulse pressure envelope, or

- from the beginning of the step change of PEEP or MPaw until the first derivative of the pulse pressure envelope reaches zero again after the maximum (time from 15 t(3-2) to 16 t(D)) or reaches zero again after the minimum (time 12 t(1-2) to 14 t(F)).

This results in the equations

$$\text{TTcp mean = t(D) - t(3-2),}$$

and

$$\text{TTcp mean = t(F) - t(1-2),}$$

wherein t(3-2) is the moment 15 of change from the highest level 10 of PEEP or MPaw (PEEP 3) to mean positive airway pressure level or the intermediate level 17 of PEEP or MPaw (PEEP 2), t(1-2) is the moment 12 of change from the lowest level 11 of PEEP or MPaw (PEEP 1) to average positive airway pressure level or the intermediate level 17 of PEEP or MPaw (PEEP 2), t(D) is the time point 16 where the envelope of arterial pulse pressure curve 5 has adapted to intermediate PEEP or MPaw level 17, and t(F) is the time point 14 where the envelope of arterial pulse pressure curve 5 has adapted to intermediate PEEP or MPaw level 17.

Using above mean transit time TTcp mean, the apparatus is adapted to calculate the mean cardiopulmonary blood volume CPBVmean by solving the equation

$$CPBVmean\ =\ COmean\ *\ TTcp\ mean,$$

wherein COmean is the mean cardiac output CO in the mean positive airway pressure phase after stabilization.

The mean left heart blood volume LHVmean can be calculated only during step changes where blood is squeezed out of the lungs, which happens only with an increase of intrathoracic pressure with increasing PEEP or MPaw. Thus, the apparatus is capable of calculating the mean left heart blood volume LHVmean by solving the equation

$$LHVmean\ =\ COmean\ *\ TTlh,mean,$$

wherein the apparatus is adapted to calculate the mean transit time TTlh,mean by solving the equation

$$TTlh,mean\ =\ t(1-2)\ -\ t(E)$$

wherein t(E) is the time point 13 where envelope curve of arterial pulse pressure 5 starts to rise.

In conventional ventilators BIPAP ventilation/respiration is a common mode. Since this mode has got only two PEEP or MPaw levels, a high PEEP or MPaw level 10 (PEEP 3) and a low PEEP or MPaw level 11 (PEEP 1), a respective mean airway pressure cannot be set. In this mode the mean cardiopulmonary blood volume CPBVmean can be continuously estimated as floating average from (CPBVPEEP1 + CPBVPEEP3)/2 or (CPBVMPaw1 + CPBVMPaw3)/2 in the same way as described above.

With regard to the left heart blood volume LHV, only LHVPEEP3 is obtained directly. However, assuming a parallel change of the cardiopulmonary blood volume CPBV and the left heart blood volume LHV during changes in PEEP or MPaw levels, the mean cardiopulmonary blood volume LHVmean can be estimated by multiplying LHVPEEP3 with the ratio CPBVmean/CPBVPEEP3.

Further, the apparatus is adapted to derive the parameters GEF and LHEF by making use of the equations

$$GEF\ =\ 4\ *\ SV/CPBV\ *\ K,$$

and

$$LHEF\ =\ 2\ *\ SV/LHV\ *\ K,$$

respectively. Where GEF is the Global Ejection Fraction, LHEF is the Left Heart Ejection Fraction. Coefficient K is an empirical correction factors to adjust GEF and LHEF to the values obtained from transpulmonary double indicator thermal dye dilution measurements.

Further, the apparatus is adapted to derive the slope of Starling curve during TriPAP for the total heart by making use of the difference quotient built from
delta SV over delta CPBV on 3 PEEP or MPaw levels, and
for the left heart by making use of the difference quotient built from
delta SV over delta LHV on 3 PEEP or MPaw levels.

Alternatively all calculations could be performed also with systolic arterial pressure or mean arterial pressure instead of pulse pressure. Alternatively in all calculations the airway pressure could be replaced by the central venous pressure or a surrogate of intrathoracic pressure like esophageal pressure or an electrical bioimpedance respiration signal.

Taking above mentioned and described modelling into account, a process for determining a patient's volemic status comprises the steps of:

-   Generating data of a physiological heart-lung interaction during spontaneous breathing or mechanical ventilation.

-   Determining the patient's volemic status when making use of the data of the physiological heart-lung interaction.

- Providing an envelope of the arterial pulse pressure 5.

- Determining the physiological heart-lung interaction by making use of the envelope of the arterial pulse pressure 5.

- Deriving the expiratory cardiopulmonary blood volume CPBVex by making use of the equation

$$CPBVex = CO * TTcp,ex,$$

wherein CO is the cardiac output and TTcp,ex is the cardiopulmonary transit time of blood in the hemodynamic status of expiration being derived from the envelope of the arterial pulse pressure 5.

- Deriving the inspiratory left heart volume LHVin by making use of the equation

$$LHVin = CO * TTlh,in,$$

wherein CO is the cardiac output and TTih,in is the inspiratory transit time of blood through the left heart being derived from the envelope of the arterial pulse pressure 5.

- Deriving the cardiopulmonary transit time of blood in the hemodynamic status of expiration TTcp,ex by making use of the equation

$$TTcp,ex = t(B) - t(I-E),$$

wherein t(I-E) is the time point 8 of end-inspiration and start of expiration, and t(B) is the time point 9 where the envelope of arterial pressure 5 reaches the same level as at the time point 6 of end-expiration and start of inspiration.

- Deriving the inspiratory transit time TTlh,in by making use of the equation

$$TTlh,in = t(E-I) - t(A),$$

wherein t(E-I) is the time point 6 of end-expiration and start of inspiration, and t(A) is the time point 7 where the envelope of arterial pressure 5 starts to rise.

- Obtaining the cardiac output CO from a continuous real time cardiac output measurement method like arterial pulse contour analysis, esophageal Doppler, transthoracic or esophageal echo Doppler, transthoracic or esophageal electrical Bioimpedance.

- Initially checking the equilibrium in the cardiopulmonary vascular system by a single extended breathing cycle in investigating as to whether a constant plateau of pulse pressure for expiration is reached in order to necessarily adjust the breathing cycle to a degree with approximate equilibrium.

- Applying the checking of equilibrium in a pressure controlled ventilation mode or in a volume controlled ventilation mode.

Alternatively, a process for determining a patient's volemic status comprises the step of:

- Deriving a middle expiratory cardiopulmonary blood volume CPBV by making use of the equation

$$CPBV = CO * TTcp,$$

wherein CO is the cardiac output and TTcp is middle cardiopulmonary transit time ranging between the cardiopulmonary transit time of blood TTcp,ex in the hemodynamic status of expiration, and the inspiratory transit time TTlh,in of blood through the left heart, both being derived from the envelope of the arterial pulse pressure 5.

Alternatively, a process for determining a patient's volemic status comprises the step of:

- Using prolonged step changes of the level of Positive End-Expiratory Pressure PEEP, or using prolonged step changes of the level of Positive End-Expiratory Pressure PEEP by breathing on three different mean airway pressure levels MPaw.

- Composing a phase of low PEEP level (PEEP 1) 11, a phase of high PEEP level (PEEP 3) 10, and a phase of intermediate PEEP level (PEEP 2) 17.

- Composing the phase of intermediate PEEP level (PEEP 2) 17 corresponding to the mean airway pressure Paw mean before a testing phase PEEP 2.

- Deriving the mean cardiopulmonary blood volume CPBVmean by making use of the equation

$$\text{CPBVmean} = \text{COmean} * \text{TTcp mean,}$$

wherein COmean is the mean cardiac output CO in the mean positive airway pressure phase after stabilization and TTcp mean is the mean cardiopulmonary transit time of blood being derived from the envelope of the arterial pulse pressure 5.

- Deriving the mean cardiopulmonary transit time of blood TTcp mean by making use of the equations

$$\text{TTcp mean} = t(D) - t(3-2),$$

or

$$\text{TTcp mean} = t(F) - t(1-2),$$

wherein t(3-2) is the moment 15 of change from the highest level 10 of PEEP or MPaw PEEP 3 to mean positive airway pressure level or the intermediate level 17 of PEEP or MPaw PEEP 2, t(1-2) is the moment 12 of change from the lowest level 11 of PEEP or MPaw PEEP 1 to average positive airway pressure level or the intermediate level 17 of PEEP or MPaw PEEP 2, t(D) is the time point 16 where the envelope of arterial pulse pressure curve 5 has adapted to intermediate PEEP or MPaw level 17, and t(F) is the time point 14 where the envelope of arterial pulse pressure curve 5 has adapted to intermediate PEEP or MPaw level 17.

- Deriving the mean left heart volume LHVmean by making use of the equation

$$\text{LHVmean} = \text{COmean} * \text{TTlh,mean,}$$

wherein COmean is the mean cardiac output CO in the mean positive airway pressure phase after stabilization and TTlh,mean is the mean transit time of blood being derived from the envelope of the arterial pulse pressure 5.

- Deriving the mean transit time of blood TTlh,mean by making use of the equation

$$\text{TTlh,mean} = t(1-2) - t(E)$$

wherein t(E) is the time point 13 where envelope curve of arterial pulse pressure 5 starts to rise.

- Deriving the slope of Starling curve during TriPAP for the total heart by making use of the difference quotient built from

delta SV over delta CPBV on 3 PEEP or MPaw levels, and
for the left heart by making use of the difference quotient built from
delta SV over delta LHV on 3 PEEP or MPaw levels.

**Claims**

1. Apparatus for determining a patient's or mammalian animal's volemic status, adapted to make use of a physiological heart-lung interaction during spontaneous breathing or mechanical ventilation,
   wherein the apparatus is configured to obtain measurements of cardiac output (CO), arterial pulse pressure (4) and airway pressure (1);
   wherein the apparatus is configured to provide an envelope of the arterial pulse pressure or a surrogate (5) and is configured to determine the physiological heart-lung interaction by making use of the envelope of the arterial pulse pressure (5),
   **characterized in that** the apparatus is configured to derive the expiratory cardiopulmonary blood volume (CPBVex) by making use of the equation

$$CPBVex = CO * TTcp,ex,$$

   wherein CO is the cardiac output and TTcp,ex is the cardiopulmonary transit time of blood in the hemodynamic status of expiration being derived from the envelope of the arterial pulse pressure (5),
   wherein the apparatus is further configured to derive the cardiopulmonary transit time of blood in the hemodynamic status of expiration (TTcp,ex) by making use of the equation

$$TTcp,ex = t(B) - t(I\text{-}E),$$

   wherein t(I-E) is the time point (8) of end-inspiration and start of expiration, and t(B) is the time point (9) where the envelope of arterial pressure (5) reaches the same level as at the time point (6) of end-expiration and start of inspiration.

2. Apparatus according to claim 1, wherein the apparatus is configured to derive the inspiratory left heart volume (LHVin) by making use of the equation

$$LHVin = CO * TTlh,in,$$

   wherein CO is the cardiac output and TTlh,in is the inspiratory transit time of blood through the left heart being derived from the envelope of the arterial pulse pressure (5), wherein the apparatus is capable to derive inspiratory transit time (TTlh,in) by making use of the equation

$$TTlh,in = t(E\text{-}I) - t(A),$$

   wherein t(E-I) is the time point (6) of end-expiration and start of inspiration, and t(A) is the time point (7) where the envelope of arterial pressure (5) starts to rise.

3. Apparatus according to claim 2, wherein the apparatus is configured to derive a middle expiratory cardiopulmonary blood volume (CPBV) by making use of the equation

$$CPBV = CO * TTcp,$$

   wherein CO is the cardiac output and TTcp is middle cardiopulmonary transit time ranging between the cardiopulmonary transit time of blood (TTcp,ex) in the hemodynamic status of expiration, and the inspiratory transit time

(TTlh,in) of blood through the left heart, both being derived from the envelope of the arterial pulse pressure (5).

4. Apparatus according to any of claims 1 to 3, wherein the apparatus is configured to obtain the cardiac output (CO) from any continuous real time cardiac output measurement method like arterial pulse contour analysis, esophageal Doppler, transthoracic or esophageal echo Doppler, transthoracic or esophageal electrical Bioimpedance, continuous heating right heart catheter,or CO2-rebreathing.

5. Apparatus according to any of claims 1 to 4, wherein the apparatus is configured to initially check the equilibrium in the cardiopulmonary vascular system by a single extended breathing cycle in investigating as to whether a constant plateau of pulse pressure for expiration is reached in order to necessarily adjust the breathing cycle to a degree with approximate equilibrium.

6. Apparatus according to claim 5, wherein the apparatus is configured to apply the checking of equilibrium in a pressure controlled ventilation mode or in a volume controlled ventilation mode.

7. Apparatus according to claim 1, wherein the apparatus is configured to use prolonged step changes of the level of Positive End-Expiratory Pressure (PEEP).

8. Apparatus according to claim 1, wherein the apparatus is configured to use prolonged step changes of the level of Positive End-Expiratory Pressure (PEEP) by breathing on three different mean airway pressure levels (MPaw).

9. Apparatus according to claim 8, wherein the apparatus is configured to compose a phase of low PEEP level (PEEP 1, 11), a phase of high PEEP level (PEEP 3, 10), and a phase of intermediate PEEP level (PEEP 2, 17).

10. Apparatus according to claim 9, wherein the apparatus is configured to compose the phase of intermediate PEEP level (PEEP 2, 17) corresponding to the mean airway pressure (Paw mean) before a testing phase (PEEP 2).

11. Apparatus according to claim 9 or claim 10, wherein the apparatus is configured to derive the mean cardiopulmonary blood volume (CPBVmean) by making use of the equation

$$CPBVmean = COmean * TTcp\ mean,$$

wherein COmean is the mean cardiac output (CO) in the mean positive airway pressure phase after stabilization and TTcp mean is the mean cardiopulmonary transit time of blood being derived from the envelope of the arterial pulse pressure (5),
wherein the apparatus is configured to derive the mean cardiopulmonary transit time of blood (TTcp mean) by making use of the equations

$$TTcp\ mean = t(D) - t(3\text{-}2),$$

or

$$TTcp\ mean = t(F) - t(1\text{-}2),$$

wherein t(3-2) is the moment (15) of change from the highest level (10) of PEEP or MPaw (PEEP 3) to mean positive airway pressure level or the intermediate level (17) of PEEP or MPaw (PEEP 2), t(1-2) is the moment (12) of change from the lowest level (11) of PEEP or MPaw (PEEP 1) to average positive airway pressure level or the intermediate level (17) of PEEP or MPaw (PEEP 2), t(D) is the time point (16) where the envelope of arterial pulse pressure curve (5) has adapted to intermediate PEEP or MPaw level (17), and t(F) is the time point (14) where the envelope of arterial pulse pressure curve (5) has adapted to intermediate PEEP or MPaw level (17).

12. Apparatus according to any of claims 9 to 11, wherein the apparatus is configured to derive the mean left heart volume (LHVmean) by making use of the equation

$$LHVmean = COmean * TTlh,mean,$$

wherein COmean is the mean cardiac output (CO) in the mean positive airway pressure phase after stabilization and TTlh,mean is the mean transit time of blood being derived from the envelope of the arterial pulse pressure (5), wherein the apparatus is configured to derive the mean transit time of blood (TTlh,mean) by making use of the equation

$$TTlh,mean = t(1-2) - t(E)$$

wherein t(E) is the time point (13) where envelope curve of arterial pulse pressure (5) starts to rise.

13. Apparatus according to any of claims 11 or 12, wherein the apparatus is configured to derive the slope of Starling curve during TriPAP for the total heart by making use of the difference quotient built from delta SV over delta CPBV on 3 PEEP or MPaw levels, and for the left heart by making use of the difference quotient built from delta SV over delta LHV on 3 PEEP or MPaw levels.

14. Computer program for determining a patient's volemic status, having instructions adapted to carry out the steps:

- obtaining measurements of arterial pulse pressure (4), airway pressure (1) and cardiac output (CO),
- generating data of a physiological heart-lung interaction during spontaneous breathing or mechanical ventilation,
- providing an envelope of the arterial pulse pressure (5) or a surrogate,
- determining the physiological heart-lung interaction by making use of the envelope of the arterial pulse pressure (5),

when run on a computer, and
**characterized by** further instructions adapted to carry out the steps:

- deriving the expiratory cardiopulmonary blood volume (CPBVex) by making use of the equation

$$CPBVex = CO * TTcp,ex,$$

wherein CO is the cardiac output and TTcp,ex is the cardiopulmonary transit time of blood in the hemodynamic status of expiration being derived from the envelope of the arterial pulse pressure (5), and
- deriving the cardiopulmonary transit time of blood in the hemodynamic status of expiration (TTcp,ex) by making use of the equation

$$TTcp,ex = t(B) - t(I-E),$$

wherein t(I-E) is the time point (8) of end-inspiration and start of expiration, and t(B) is the time point (9) where the envelope of arterial pressure (5) reaches the same level as at the time point (6) of end-expiration and start of inspiration.

15. Computer program according to claim 14, having instructions adapted to carry out the step:

- deriving the inspiratory left heart volume (LHVin) by making use of the equation

$$LHVin = CO * TTlh,in,$$

wherein CO is the cardiac output and TTlh,in is the inspiratory transit time of blood through the left heart being derived from the envelope of the arterial pulse pressure (5), and
- deriving the inspiratory transit time (TTlh,in) by making use of the equation

$$TTlh,in = t(E-I) – t(A),$$

wherein t(E-I) is the time point (6) of end-expiration and start of inspiration, and t(A) is the time point (7) where the envelope of arterial pressure (5) starts to rise.

**16.** Computer program according to claim 14, having instructions adapted to carry out the step:

- deriving a middle expiratory cardiopulmonary blood volume (CPBV) by making use of the equation

$$CPBV = CO * TTcp,$$

wherein CO is the cardiac output and TTcp is middle cardiopulmonary transit time ranging between the cardiopulmonary transit time of blood (TTcp,ex) in the hemodynamic status of expiration, and the inspiratory transit time (TTlh,in) of blood through the left heart, both being derived from the envelope of the arterial pulse pressure (5).

**17.** Computer program according to any of claims 14 to 16, having instructions adapted to carry out the step:

- obtaining the cardiac output (CO) from a continuous real time cardiac output measurement method like arterial pulse contour analysis, esophageal Doppler, transthoracic or esophageal echo Doppler, transthoracic or esophageal electrical Bioimpedance.

**18.** Computer program according to any of claims 14 to 17, having instructions adapted to carry out the step:

- initially checking the equilibrium in the cardiopulmonary vascular system by a single extended breathing cycle in investigating as to whether a constant plateau of pulse pressure for expiration is reached in order to necessarily adjust the breathing cycle to a degree with approximate equilibrium.

**19.** Computer program according to claim 18, having instructions adapted to carry out the step:

- applying the checking of equilibrium in a pressure controlled ventilation mode or in a volume controlled ventilation mode.

**20.** Computer program according to claim 14, having instructions adapted to carry out the step:

- using prolonged step changes of the level of Positive End-Expiratory Pressure (PEEP).

**21.** Computer program according to claim 14, having instructions adapted to carry out the step:

- using prolonged step changes of the level of Positive End-Expiratory Pressure (PEEP) by breathing on three different mean airway pressure levels (MPaw).

**22.** Computer program according to claim 21, having instructions adapted to carry out the step:

- composing a phase of low PEEP level (PEEP 1, 11), a phase of high PEEP level (PEEP 3, 10), and a phase of intermediate PEEP level (PEEP 2, 17).

**23.** Computer program according to claim 22, having instructions adapted to carry out the step:

- composing the phase of intermediate PEEP level (PEEP 2, 17) corresponding to the mean airway pressure (Paw mean) before a testing phase (PEEP 2).

**24.** Computer program according to claim 22 or 23, having instructions adapted to carry out the step:

- deriving the mean cardiopulmonary blood volume (CPBVmean) by making use of the equation

$$CPBVmean = COmean * TTcp\ mean,$$

wherein COmean is the mean cardiac output (CO) in the mean positive airway pressure phase after stabilization and TTcp mean is the mean cardiopulmonary transit time of blood being derived from the envelope of the arterial pulse pressure (5), and
- deriving the mean cardiopulmonary transit time of blood (TTcp mean) by making use of the equations

$$TTcp\ mean = t(D) - t(3-2),$$

or

$$TTcp\ mean = t(F) - t(1-2),$$

wherein t(3-2) is the moment (15) of change from the highest level (10) of PEEP or MPaw (PEEP 3) to mean positive airway pressure level or the intermediate level (17) of PEEP or MPaw (PEEP 2), t(1-2) is the moment (12) of change from the lowest level (11) of PEEP or MPaw (PEEP 1) to average positive airway pressure level or the intermediate level (17) of PEEP or MPaw (PEEP 2), t(D) is the time point (16) where the envelope of arterial pulse pressure curve (5) has adapted to intermediate PEEP or MPaw level (17), and t(F) is the time point (14) where the envelope of arterial pulse pressure curve (5) has adapted to intermediate PEEP or MPaw level (17).

25. Computer program according to any of claims 22 to 24, having instructions adapted to carry out the step:

- deriving the mean left heart volume (LHVmean) by making use of the equation

$$LHVmean = COmean * TTlh,mean,$$

wherein COmean is the mean cardiac output (CO) in the mean positive airway pressure phase after stabilization and TTlh,mean is the mean transit time of blood being derived from the envelope of the arterial pulse pressure (5), and
- deriving the mean transit time of blood (TTlh,mean) by making use of the equation

$$TTlh,mean = t(1-2) - t(E)$$

wherein t(E) is the time point (13) where envelope curve of arterial pulse pressure (5) starts to rise.

26. Computer program according to any of claims 24 or 25, having instructions adapted to carry out the step:

- deriving the slope of Starling curve during TriPAP for the total heart by making use of the difference quotient built from delta SV over delta CPBV on at least two PEEP or MPaw levels, and for the left heart by making use of the difference quotient built from delta SV over delta LHV on at least two PEEP or MPaw levels.

**Patentansprüche**

1. Vorrichtung zum Bestimmen des volemischen Status eines Patienten oder eines Säugetieres, welche in der Lage ist, während einer Spontanatmung oder einer mechanischen Beatmung von einer physiologischen Herz-Lungen-Interaktion Gebrauch zu machen,
wobei die Vorrichtung ausgebildet ist, Messungen der Herzleistung (CO), des arteriellen Pulsdruckes (4) und des Luftwegedruckes (1) zu erhalten;
wobei die Vorrichtung ausgebildet ist, eine Hüllkurve des arteriellen Pulsdruckes oder eines Surrogats (5) bereitzustellen, und wobei die Vorrichtung ausgebildet ist, die physiologische Herz-Lungen-Interaktion durch Nutzung der Hüllkurve des arteriellen Pulsdruckes (5) zu bestimmen,

**dadurch gekennzeichnet, dass** die Vorrichtung ausgebildet ist, das kardiopulmonare Ausatmungsblutvolumen (CPBVex) unter Verwendung der Gleichung

$$CPBVex = CO \cdot TTcp,ex,$$

abzuleiten,

wobei CO die Herzleistung und TTcp,ex die kardiopulmonare Übergangszeit des Blutes in den hämodynamischen Status der Ausatmung, welche aus der Hüllkurve des arteriellen Pulsdruckes (5) erhalten wird, ist,

wobei die Vorrichtung weiterhin ausgebildet ist, die kardiopulmonare Übergangszeit des Blutes in den hämodynamischen Status der Ausatmung (TTcp,ex) unter Verwendung der Gleichung

$$TTcp,ex = t(B) - t(I\text{-}E),$$

abzuleiten,

wobei t(I-E) der Zeitpunkt (8) des Endes der Einatmung sowie der Beginn der Ausatmung und t(B) der Zeitpunkt (9) ist, zu dem die Hüllkurve des arteriellen Druckes (5) das gleiche Niveau wie zu dem Zeitpunkt (6) des Endes der Ausatmung und des Beginns der Einatmung erreicht.

2. Vorrichtung nach Anspruch 1,
wobei die Vorrichtung ausgebildet ist, das inspiratorische linke Herzvolumen (LHVin) unter Verwendung der Gleichung

$$LHVin = CO \cdot TTlh,in$$

abzuleiten,

wobei CO die Herzleistung und TTlh,in die inspiratorische Übergangszeit des Blutes durch das linke Herz ist, welche aus der Hüllkurve des arteriellen Pulsdruckes (5) erhalten wird, wobei die Vorrichtung in der Lage ist, die inspiratorische Übergangszeit (TTlh,in) unter Verwendung der Gleichung

$$TTlh,in = t(E\text{-}I) - t(A)$$

abzuleiten,

wobei t(E-I) der Zeitpunkt (6) des Endes der Ausatmung und der Beginn der Einatmung sowie t(A) der Zeitpunkt (7) ist, bei dem die Hüllkurve des arteriellen Druckes (5) beginnt anzusteigen.

3. Vorrichtung nach Anspruch 2,
wobei die Vorrichtung ausgebildet ist, ein mittleres expiratorisches kardiopulmonares Blutvolumen (CPBV) unter Verwendung der Gleichung

$$CPBV = CO \cdot TTcp$$

abzuleiten,

wobei CO die Herzleistung und TTcp die mittlere kardiopulmonare Übergangszeit ist, welche sich zwischen der kardiopulmonaren Übergangszeit des Blutes (TTcp,ex) in den hämodynamischen Status der Ausatmung und der inspiratorischen Übergangszeit (TTlh,in) des Blutes durch das linke Herz erstreckt, wobei beide aus der Hüllkurve des arteriellen Pulsdruckes (5) abgeleitet werden.

4. Vorrichtung nach einem der Ansprüche 1 bis 3,
wobei die Vorrichtung ausgebildet ist, die Herzleistung (CO) von jedem kontinuierlichen Echtzeit-Herzleistungsmessverfahren, wie die arterielle Pulskontouranalyse, den Oesophagus-Doppler, den transthorakalen oder oesophagalen Echodoppler, die transthorakale oder oesophagalelektrische Bioimpedanz, den kontinuierlich erhitzten rechten Herzkatheter oder die $CO_2$-Rückatmung zu erhalten.

5. Vorrichtung nach einem der Ansprüche 1 bis 4,
wobei die Vorrichtung ausgebildet ist, zu Beginn das Gleichgewicht in dem kardiopulmonaren Vaskularsystem durch einen einzelnen erweiterten Atmungszyklus mittels Untersuchung, ob ein konstantes Plateau des Pulsdruckes für die Ausatmung erreicht wird, um den Atmungszyklus auf einen Grad mit annäherndem Gleichgewicht notwendigerweise einzustellen, zu prüfen.

6. Vorrichtung nach Anspruch 5,
wobei die Vorrichtung ausgebildet ist, die Überprüfung des Gleichgewichts in einem durchgesteuerten Beatmungsmodus oder in einem volumengesteuerten Beatmungsmodus, anzuwenden.

7. Vorrichtung nach Anspruch 1,
wobei die Vorrichtung ausgebildet ist, verlängerte Schrittänderungen des Niveaus des positiven End-Ausatmungsdruckes (PEEP) zu verwenden.

8. Vorrichtung nach Anspruch 1,
wobei die Vorrichtung ausgebildet ist, verlängerte Schrittänderungen des Niveaus des positiven End-Ausatmungsdruckes (PEEP) durch Atmen auf drei unterschiedlichen mittleren Luftweg-Druckniveaus (MPaw) zu verwenden.

9. Vorrichtung nach Anspruch 8,
wobei die Vorrichtung ausgebildet ist, eine Phase des niedrigen PEEP-Niveaus (PEEP 1, 11), eine Phase des hohen PEEP-Niveaus (PEEP 3, 10) und eine Phase eines Zwischen-PEEP-Niveaus (PEEP 2, 17) zu bilden.

10. Vorrichtung nach Anspruch 9,
wobei die Vorrichtung ausgebildet ist, die Phase des Zwischen-PEEP-Niveaus (PEEP 2, 17) entsprechend dem mittleren Luftwegdruck (Paw mean) vor einer Testphase (PEEP 2) zu bilden.

11. Vorrichtung nach Anspruch 9 oder Anspruch 10,
wobei die Vorrichtung ausgebildet ist, das mittlere kardiopulmonare Blutvolumen (CPBVmean) unter Verwendung der Gleichung

$$CPBVmean = COmean \cdot TTcp\ mean$$

abzuleiten,
wobei COmean die mittlere Herzleistung (CO) in der mittleren positiven Luftwegdruckphase nach Stabilisierung und TTcp mean die mittlere kardiopulmonare Übergangszeit des Blutes ist, welche aus der Hüllkurve des arteriellen Pulsdruckes (5) abgeleitet worden ist,
wobei die Vorrichtung ausgebildet ist, die mittlere kardiopulmonare Übergangszeit des Blutes (TTcp mean) unter Verwendung der Gleichungen

$$TTcp\ mean = t(D) - t(3\text{-}2)$$

oder

$$TTcp\ mean = t(F) - t(1\text{-}2)$$

abzuleiten,
wobei t(3-2) der Augenblick (15) der Änderung von dem höchsten Niveau (10) des PEEP oder des MPaw (PEEP 3) zu dem mittleren positiven Luftweg-Druckniveau oder das Zwischenniveau (17) des PEEP oder des MPaw (PEEP 2) ist, t(1-2) der Augenblick (12) der Änderung von dem niedrigsten Niveau (11) des PEEP oder des MPaw (PEEP 1) zum durchschnittlichen positiven Luftweg-Druckniveau oder des Zwischenniveaus (17) des PEEP oder des MPaw (PEEP 2) ist, t(D) der Zeitpunkt (16) ist, bei dem die Hüllkurve der arteriellen Pulsdruckkurve (5) auf das mittlere PEEP- oder MPaw-Niveau (17) angepasst ist, und t(F) der Zeitpunkt (14) ist, bei dem die Hüllkurve der arteriellen Pulsdruckkurve (5) auf das mittlere PEEP- oder MPaw-Niveau (17) angepasst ist.

12. Vorrichtung nach einem der Ansprüche 9 bis 11,
wobei die Vorrichtung ausgebildet ist, das mittlere linke Herzvolumen (LHVmean) unter Verwendung der Gleichung

$$LHVmean = COmean \cdot TTlh,mean$$

abzuleiten,
wobei COmean die mittlere Herzleistung (CO) in der mittleren positiven Luftweg-Druckphase nach Stabilisierung und TTlh,mean die mittlere Übergangszeit des Blutes ist, welche aus der Hüllkurve des arteriellen Pulsdruckes (5) erhalten wird,
wobei die Vorrichtung ausgebildet ist, die mittlere Übergangszeit des Blutes (TTlh,mean) unter Verwendung der Gleichung

$$TTlh,mean = t(1\text{-}2) - t(E)$$

abzuleiten,
wobei t(E) der Zeitpunkt (13) ist, bei dem die Hüllkurve des arteriellen Pulsdruckes (5) beginnt anzusteigen.

13. Vorrichtung nach einem der Ansprüche 11 oder 12,
wobei die Vorrichtung ausgebildet ist, die Neigung der Starling-Kurve während TriPAP für das gesamte Herz unter Verwendung des Differenzquotientens, welcher aus delta SV über delta CPBV auf 3-PEEP- oder MPaw-Niveaus gebildet wird, und für das linke Herz unter Verwendung des Differenzquotientens, welcher aus delta SV über delta LHV auf 3-PEEP- oder MPaw-Niveaus gebildet wird, abzuleiten.

14. Computerprogramm zum Bestimmen des volemischen Status eines Patienten mit Befehlen, die in der Lage sind, die folgenden Schritte auszuführen:

- Erhalten von Messungen des arteriellen Pulsdruckes (4), des Luftwegdruckes (1) und der Herzleistung (CO),
- Erzeugen von Daten einer physiologischen Herz-Lungen-Interaktion während einer Spontanatmung oder einer mechanischen Beatmung,
- Bereitstellen einer Hüllkurve des arteriellen Pulsdrucks (5) oder eines Surrogats,
- Bestimmen der physiologischen Herz-Lungen-Interaktion unter Verwendung der Hüllkurve des arteriellen Pulsdruckes (5),

wenn diese auf einem Computer verarbeitet werden, und
**gekennzeichnet durch** die weiteren Befehle, welche in der Lage sind, die folgenden Schritte auszuführen:

- Ableiten des expiratorischen kardiopulmonaren Blutvolumens (CPBVex) unter Verwendung der Gleichung

$$CPBVex = CO \cdot TTcp,ex,$$

wobei CO die Herzleistung und TTcp,ex die kardiopulmonare Übergangszeit des Blutes in den hämodynamischen Status des Ausatmens, welche aus der Hüllkurve des arteriellen Pulsdruckes (5) erhalten wird, ist, und
- Ableiten der kardiopulmonaren Übergangszeit des Blutes in den hämodynamischen Status der Ausatmung (TTcp,ex) unter Verwendung der Gleichung

$$TTcp,ex = t(B) - t(I\text{-}E),$$

wobei t(I-E) der Zeitpunkt (8) des Endes der Einatmung sowie der Beginn der Ausatmung ist und t(B) der Zeitpunkt (9) ist, bei dem die Hüllkurve des arteriellen Druckes (5) das gleiche Niveau wie zu dem Zeitpunkt (6) des Endes der Ausatmung und des Beginns der Einatmung erreicht.

**15.** Computerprogramm nach Anspruch 14 mit Befehlen, die geeignet sind, den folgenden Schritt auszuführen:

- Ableiten des inspiratorischen linken Herzvolumens (LHVin) unter Verwendung der Gleichung

$$\text{LHVin} = \text{CO} \cdot \text{TTlh,in,}$$

wobei CO die Herzleistung und TTlh,in die Einatmungsübergangszeit des Blutes des linken Herzes ist, welche aus der Hüllkurve des arteriellen Pulsdruckes (5) abgeleitet wird, und
- Ableiten der Einatmungsübergangszeit (TTlh,in) unter Verwendung der Gleichung

$$\text{TTlh,in} = t(E\text{-}I) - t(A),$$

wobei t(E-I) der Zeitpunkt (6) des Endes der Ausatmung und des Beginns der Einatmung ist und t(A) der Zeitpunkt (7) ist, bei dem die Hüllkurve des arteriellen Druckes (5) beginnt anzusteigen.

**16.** Computerprogramm nach Anspruch 14 mit Befehlen, die ausgebildet sind, den folgenden Schritt auszuführen:

- Ableiten eines mittleren kardiopulmonaren expiratorischen Blutvolumens (CPBV) unter Verwendung der Gleichung

$$\text{CPBV} = \text{CO} \cdot \text{TTcp,}$$

wobei CO die Herzleistung und TTcp die mittlere kardiopulmonare Übergangszeit ist, die sich zwischen der kardiopulmonaren Übergangszeit des Blutes (TTcp,ex) in den hämodynamischen Status der Ausatmung und der Einatmungsübergangszeit (TTlh,in) des Blutes durch das linke Herz erstreckt, wobei beide aus der Hüllkurve des arteriellen Pulsdruckes (5) erhalten werden.

**17.** Computerprogramm nach einem der Ansprüche 14 bis 16 mit Befehlen, die ausgebildet sind, den folgenden Schritt auszuführen:

- Erhalten der Herzleistung (CO) aus einem kontinuierlichen Echtzeit-Herzleistungsmessverfahren, wie die arterielle Pulskontouranalyse, den Oesophagus-Doppler, den transthorakalen oder oesophagalen Echodoppler, die transthorakale oder oesophagalelektrische Bioimpedanz.

**18.** Computerprogramm nach einem der Ansprüche 14 bis 17 mit Befehlen, die ausgebildet sind, den folgenden Schritt auszuführen:

- zu Beginn Überprüfen des Gleichgewichtes in dem kardiopulmonaren Vaskularsystem durch einen einzelnen, erweiterten Atmungszyklus mittels Untersuchung, ob ein konstantes Plateau des Pulsdruckes für die Ausatmung erreicht wird, um den Atmungszyklus auf einen Grad mit angenähertem Gleichgewicht notwendigerweise anzupassen.

**19.** Computerprogramm nach Anspruch 18 mit Befehlen, die geeignet sind, den folgenden Schritt auszuführen:

- Anwenden der Überprüfung des Gleichgewichtes in einem druckkontrollierten Beatmungsmodus oder in einem volumenkontrollieren Beatmungsmodus.

**20.** Computerprogramm nach Anspruch 14 mit Befehlen, die geeignet sind, den folgenden Schritt auszuführen:

- Verwenden verlängerter Schrittwechsel des Niveaus des positiven Endausatmungsdruckes (PEEP).

**21.** Computerprogramm nach Anspruch 14 mit Befehlen, die geeignet sind, den folgenden Schritt auszuführen:

- Verwendung verlängerter Schrittänderungen des Niveaus des positiven Endausatmungsdruckes (PEEP) durch Beatmung auf drei verschiedenen mittleren Luftdruckniveaus (MPaw).

22. Computerprogramm nach Anspruch 21 mit Befehlen, die geeignet sind, den folgenden Schritt auszuführen:

- Bilden einer Phase eines niedrigen PEEP-Niveaus (PEEP 1, 11), einer Phase eines hohen PEEP-Niveaus (PEEP 3, 10) und einer Phase eines Zwischen-PEEP-Niveaus (PEEP 2, 17).

23. Computerprogramm nach Anspruch 22 mit Befehlen, die geeignet sind, den folgenden Schritt auszuführen:

- Bilden der Phase des Zwischen-PEEP-Niveaus (PEEP 2, 17) entsprechend dem mittleren Luftwegdruck (Paw mean) vor einer Testphase (PEEP 2).

24. Computerprogramm nach Anspruch 22 oder 23 mit Befehlen, die geeignet sind, den folgenden Schritt auszuführen:

- Ableiten des mittleren kardiopulmonaren Blutvolumens (CPBVmean) unter Verwendung der Gleichung

$$CBPVmean = COmean \cdot TTcp\ mean,$$

wobei COmean die mittlere Herzleistung (CO) in der mittleren positiven Luftweg-Druckphase nach Stabilisierung und TTcp mean die mittlere kardiopulmonare Übergangszeit des Blutes ist, welche aus der Hüllkurve des arteriellen Pulsdruckes (5) erhalten wird, und
- Ableiten der mittleren kardiopulmonaren Übergangszeit des Blutes (TTcp mean) unter Verwendung der Gleichungen

$$TTcp\ mean = t(D) - t(3\text{-}2)$$

oder

$$TTcp\ mean = t(F) - t(1\text{-}2),$$

wobei t(3-2) der Augenblick (15) der Änderung von dem höchsten Niveau (10) des PEEP oder des MPaw (PEEP 3) zum mittleren positiven Luftweg-Druckniveau oder das Zwischenniveau (17) des PEEP oder des MPaw (PEEP 2) ist, t(1-2) der Augenblick (12) der Änderung von dem niedrigsten Niveau (11) des PEEP oder des MPaw (PEEP 1) zu dem durchschnittlichen positiven Luftweg-Druckniveau oder dem Zwischenniveau (17) des PEEP oder des MPaw (PEEP 2) ist, t(D) der Zeitpunkt (16) ist, bei dem die Hüllkurve der arteriellen Pulsdruckkurve (5) auf das Zwischen-PEEP- oder das MPaw-Niveau (17) angepasst ist, und t(F) der Zeitpunkt (14) ist, bei dem die Hüllkurve der arteriellen Pulsdruckkurve (5) auf das Zwischen-PEEP- oder das MPaw-Niveau (17) angepasst ist.

25. Computerprogramm nach einem der Ansprüche 22 bis 24 mit Befehlen, die geeignet sind, den folgenden Schritt auszuführen:

- Ableiten des mittleren linken Herzvolumens (LHVmean) unter Verwendung der Gleichung

$$LHVmean = COmean \cdot TTlh,mean,$$

wobei COmean die mittlere Herzleistung (CO) in der mittleren positiven Luftweg-Druckphase nach Stabilisierung und TTlh,mean die mittlere Übergangszeit des Blutes ist, welche aus der Hüllkurve des arteriellen Pulsdruckes (5) erhalten wird, und
- Ableiten der mittleren Übergangszeit des Blutes (TTlh,mean) unter Verwendung der Gleichung

$$TTlh,mean = t(1\text{-}2) - t(E),$$

wobei t(E) der Zeitpunkt (13) ist, bei dem die Hüllkurve des arteriellen Pulsdruckes (5) beginnt anzusteigen.

**26.** Computerprogramm nach einem der Ansprüche 24 oder 25 mit Befehlen, die geeignet sind, den folgenden Schritt auszuführen:

- Erhalten des Anstiegs der Starling-Kurve während TriPAP für das gesamte Herz unter Verwendung des Differenzquotientens, welcher aus delta SV über delta CPBV an zumindest zwei PEEP- oder MPaw-Niveaus erhalten wird, und für das linke Herz unter Verwendung des Differenzquotientens, welcher aus dem delta SV über delta LHV auf zumindest zwei PEEP- oder MPaw-Niveaus gebildet ist.

**Revendications**

**1.** Dispositif pour déterminer un état volémique de patient ou d'animal mammifère, adapté pour faire usage d'une interaction physiologique coeur-poumon pendant une respiration spontanée ou une ventilation mécanique, dans lequel le dispositif est configuré pour obtenir des mesures du débit cardiaque (CO), de la pression différentielle artérielle (4) et de la pression dans les voies aériennes (1); dans lequel le dispositif est configuré pour fournir une enveloppe de la pression différentielle artérielle ou un substitut (5) et est configuré pour déterminer l'interaction physiologique coeur-poumon, en faisant usage de l'enveloppe de la pression différentielle artérielle (5), **caractérisé en ce que** le dispositif est configuré pour déduire le volume sanguin cardiopulmonaire expiratoire (CPBVex), en faisant usage de l'équation

$$CPBVex = CO*TTcp,ex,$$

dans laquelle CO est le débit cardiaque et TTcp,ex est le temps de transit cardiopulmonaire du sang dans l'état hémodynamique d'expiration déduit de l'enveloppe de la pression différentielle artérielle (5), dans lequel le dispositif est configuré, en outre, pour déduire le temps de transit cardiopulmonaire du sang dans l'état hémodynamique d'expiration (TTcp,ex), en faisant usage de l'équation

$$TTcp,ex = t(B)-t(I-E),$$

dans laquelle t(I-E) est l'instant (8) d'inspiration finale et de début d'expiration et t(B) est l'instant (9) où l'enveloppe de la pression artérielle (5) atteint le même niveau qu'à l'instant (6) d'expiration finale et de début d'inspiration.

**2.** Dispositif suivant la revendication 1, dans lequel le dispositif est configuré pour déduire le volume inspiratoire du coeur gauche (LHVin), en faisant usage de l'équation

$$LVHin = CO*TTlh,in,$$

dans laquelle CO est le débit cardiaque et TTlh,in est le temps de transit inspiratoire du sang dans le coeur gauche déduit de l'enveloppe de la pression différentielle artérielle (5), le dispositif étant apte à déduire le temps de transit inspiratoire (TTlh,in), en faisant usage de l'équation

$$TTlh,in = t(E-I)-t(A),$$

dans laquelle t(E-I) est l'instant (6) d'expiration finale et le début d'inspiration et t(A) est l'instant (7) où l'enveloppe de la pression artérielle (5) commence à s'élever.

**3.** Dispositif suivant la revendication 2, dans lequel le dispositif est configuré pour déduire le volume sanguin cardiopulmonaire expiratoire médian (CPBV), en faisant usage de l'équation

$$CPBV = CO*TTcp,$$

dans laquelle CO est le débit cardiaque et TTcp est le temps de transit cardiopulmonaire médian compris entre le temps de transit cardiopulmonaire du sang (TTcp,ex) dans l'état hémodynamique d'expiration et le temps de transit inspiratoire (TTlh,in) du sang dans le coeur gauche, tous deux étant déduits de l'enveloppe de la pression différentielle artérielle (5).

4. Dispositif suivant l'une quelconque des revendications 1 à 3, dans lequel le dispositif est configuré pour obtenir le débit cardiaque (CO) par tout procédé de mesure du débit cardiaque continu en temps réel, comme une analyse du contour du pouls artériel, un doppler oesophagien, un doppler écho transthoracique ou oesophagien, une bioimpédance électrique transthoracique ou oesophagienne, un cathéter de coeur droit de chauffage continu ou une réinhalation de CO2.

5. Dispositif suivant l'une quelconque des revendications 1 à 4, dans lequel le dispositif est configuré pour contrôler initialement l'équilibre dans le système vasculaire cardiopulmonaire par un cycle de respiration unique prolongée, en recherchant si un plateau constant de pression différentielle pour l'expiration est atteint, afin d'ajuster nécessairement le cycle respiratoire à un degré ayant un équilibre approximatif.

6. Dispositif suivant la revendication 5, dans lequel le dispositif est configuré pour appliquer le contrôle d'équilibre dans un mode de ventilation commandé par la pression ou dans un mode de ventilation commandé par le volume.

7. Dispositif suivant la revendication 1, dans lequel le dispositif est configuré pour utiliser des changements de stade prolongés du niveau de pression expiratoire finale positive (PEEP).

8. Dispositif suivant la revendication 1, dans lequel le dispositif est configuré pour utiliser des changements de stade prolongés du niveau de pression expiratoire finale positive (PEEP) en respirant sur trois niveaux de pression moyens différents dans les voies respiratoires (MPaw).

9. Dispositif suivant la revendication 8, dans lequel le dispositif est configuré pour composer une phase de bas niveau de PEEP (PEEP 1, 11), une phase de haut niveau de PEEP (PEEP 3, 10) et une phase de niveau intermédiaire de PEEP (PEEP 2, 17).

10. Dispositif suivant la revendication 9, dans lequel le dispositif est configuré pour composer la phase de niveau intermédiaire de PEEP (PEEP 2, 17) correspondant à la pression moyenne dans les voies respiratoires (Paw mean) avant une phase de test (PEEP 2).

11. Dispositif suivant la revendication 9 ou la revendication 10, dans lequel le dispositif est configuré pour déduire le volume sanguin cardiopulmonaire moyen (CPBVmean), en faisant usage de l'équation

$$CPBVmean = COmean*TTcp\ mean,$$

dans laquelle COmean est le débit cardiaque (CO) moyen dans la phase de pression positive moyenne dans les voies respiratoires après stabilisation et TTcp mean est le temps de transit cardiopulmonaire moyen du sang déduit de l'enveloppe de la pression différentielle artérielle (5),
dans lequel le dispositif est configuré pour déduire le temps de transit cardiopulmonaire moyen du sang (TTcp mean), en faisant usage des équations

$$TTcp\ mean = t(D)-t(3-2)$$

ou

$$TTcp\ mean = t(F)-t(1-2),$$

EP 1 870 035 B1

dans lesquelles t(3-2) est l'instant (15) de passage du niveau (10) le plus haut de PEEP ou de MPaw (PEEP 3) au niveau moyen de pression positive dans les voies respiratoires ou au niveau (17) intermédiaire de PEEP ou de MPaw (PEEP 2), t(1-2) est l'instant (12) de passage du niveau (11) le plus bas de PEEP ou de MPaw (PEEP 1) au niveau moyen de pression positive dans les voies aériennes ou au niveau (17) intermédiaire de PEEP ou de MPaw (PEEP 2), t(D) est l'instant (16) où l'enveloppe de la courbe de la pression différentielle artérielle (5) s'est adaptée à la PEEP intermédiaire ou au niveau de MPaw (17) et t(F) est l'instant (14) où l'enveloppe de la courbe de la pression différentielle artérielle (5) s'est adaptée à la PEEP intermédiaire ou au niveau de MPaw (17).

**12.** Dispositif suivant l'une quelconque des revendications 9 à 11, dans lequel le dispositif est configuré pour déduire le volume moyen du coeur gauche (LHVmean), en faisant usage de l'équation

$$LHVmean = COmean*TTlh,mean,$$

dans laquelle COmean est le débit cardiaque (CO) moyen dans la phase moyenne de pression positive dans les voies aériennes après stabilisation et TTlh,mean est le temps de transit moyen du sang déduit de l'enveloppe de la pression différentielle artérielle (5),
dans lequel le dispositif est configuré pour déduire le temps de transit moyen du sang (TTlh,mean), en faisant usage de l'équation

$$TTlh,mean = t(1-2)-t(E),$$

dans laquelle t(E) est l'instant (13) où la courbe d'enveloppe de la pression différentielle artérielle (5) commence à s'élever.

**13.** Dispositif suivant l'une quelconque des revendications 11 ou 12, dans lequel le dispositif est configuré pour déduire la pente de la courbe de Starling pendant TriPAP pour le coeur total, en faisant usage du quotient de différence formé à partir de delta SV sur delta CPBV sur au moins deux niveaux de PEEP ou de MPaw et, pour le coeur gauche, en faisant usage du quotient de différence formé à partir de delta SV sur delta LHV sur au moins deux niveaux de PEEP ou de MPaw.

**14.** Programme d'ordinateur pour déterminer un état volémique d'un patient, ayant des instructions adaptées pour effectuer les stades dans lesquels :

- on obtient des mesures d'une pression différentielle artérielle (4), d'une pression dans les voies respiratoires (1) et d'un débit cardiaque (CO),
- on crée des données d'une interaction physiologique coeur-poumon pendant une respiration spontanée ou une ventilation mécanique,
- on procure une enveloppe de la pression différentielle artérielle (5) ou d'un substitut,
- on détermine l'interaction physiologique coeur-poumon, en faisant usage de l'enveloppe de la pression différentielle artérielle (5),

lorsqu'elles sont passées sur un ordinateur et
**caractérisé par** d'autres instructions adaptées pour effectuer les stades dans lesquels :

- on déduit le volume sanguin cardiopulmonaire expiratoire (CPBVex), en faisant usage de l'équation

$$CPBVex = CO*TTcp,ex,$$

dans laquelle CO est le débit cardiaque et TTcp,ex est le temps de transit cardiopulmonaire du sang dans l'état hémodynamique d'expiration déduit de l'enveloppe de la pression différentielle artérielle (5) et
- on déduit le temps de transit cardiopulmonaire du sang dans l'état hémodynamique d'expiration (TTcp,ex), en faisant usage de l'équation

24

$$TTcp,ex = t(B)-t(I-E),$$

dans laquelle t(I-E) est l'instant (8) d'inspiration finale et de début d'expiration et t(B) est l'instant (9) où l'enveloppe de la pression artérielle (5) atteint le même niveau qu'à l'instant (6) d'expiration finale et de début d'inspiration.

**15.** Programme d'ordinateur suivant la revendication 14, ayant des instructions adaptées pour effectuer le stade dans lequel :

- on déduit le volume inspiratoire du coeur gauche (LHVin), en faisant usage de l'équation

$$LVHin = CO*TTlh,in,$$

dans laquelle CO est le débit cardiaque et TTlh,in est le temps de transit inspiratoire du sang dans le coeur gauche déduit de l'enveloppe de la pression différentielle artérielle (5) et
- on déduit le temps TTlh,in de transit inspiratoire du sang, en faisant usage de l'équation

$$TTlh,in = t(E-I)-t(A),$$

dans laquelle t(E-I) est l'instant (6) d'expiration finale et le début d'inspiration et t(A) est l'instant (7) où l'enveloppe de la pression artérielle (5) commence à s'élever.

**16.** Programme d'ordinateur suivant la revendication 14, ayant des instructions adaptées pour effectuer le stade dans lequel :

- on déduit le volume sanguin cardiopulmonaire expiratoire médian (CPBV), en faisant usage de l'équation

$$CPBV = CO*TTcp,$$

dans laquelle CO est le débit cardiaque et TTcp est le temps de transit cardiopulmonaire médian compris entre le temps de transit cardiopulmonaire du sang (TTcp,ex) dans l'état hémodynamique d'expiration et le temps de transit inspiratoire (TTlh,in) du sang dans le coeur gauche, tous deux étant déduits de l'enveloppe de la pression différentielle artérielle (5).

**17.** Programme d'ordinateur suivant l'une quelconque des revendications 14 à 16, ayant des instructions adaptées pour effectuer le stade dans lequel :

- on obtient le débit cardiaque (CO) par tout procédé de mesure du débit cardiaque continu en temps réel, comme une analyse du contour du pouls artériel, un doppler oesophagien, un doppler écho transthoracique ou oesophagien, une bioimpédance électrique transthoracique ou oesophagienne.

**18.** Programme d'ordinateur suivant l'une quelconque des revendications 14 à 17, ayant des instructions adaptées pour effectuer le stade dans lequel :

- on vérifie initialement l'équilibre dans le système vasculaire cardiopulmonaire par un cycle de respiration unique prolongée, en recherchant si un plateau constant de pression différentielle pour l'expiration est atteint, afin d'ajuster nécessairement le cycle respiratoire à un degré ayant un équilibre approximatif.

**19.** Programme d'ordinateur suivant la revendication 18, ayant des instructions adaptées pour effectuer le stade dans lequel :

- on applique le contrôle d'équilibre dans un mode de ventilation commandé par la pression ou dans un mode de ventilation commandé par le volume.

**20.** Programme d'ordinateur suivant la revendication 14, ayant des instructions adaptées pour effectuer le stade dans lequel :

- on utilise des changements de stade prolongés du niveau de pression expiratoire finale positive (PEEP).

**21.** Programme d'ordinateur suivant la revendication 14, ayant des instructions adaptées pour effectuer le stade dans lequel :

- on utilise des changements de stade prolongés du niveau de pression expiratoire finale positive (PEEP) en respirant sur trois niveaux de pression ou moyens différents dans les voies respiratoires (MPaw).

**22.** Programme d'ordinateur suivant la revendication 21, ayant des instructions adaptées pour effectuer le stade dans lequel :

- on compose une phase de bas niveau de PEEP (PEEP 1, 11), une phase de haut niveau de PEEP (PEEP 3, 10) et une phase de niveau intermédiaire de PEEP (PEEP 2, 17).

**23.** Programme d'ordinateur suivant la revendication 22, ayant des instructions adaptées pour effectuer le stade dans lequel :

- on compose la phase de niveau intermédiaire de PEEP (PEEP 2, 17) correspondant à la pression moyenne dans les voies respiratoires (Paw mean) avant une phase de test (PEEP 2).

**24.** Programme d'ordinateur suivant la revendication 22 ou 23, ayant des instructions adaptées pour effectuer le stade dans lequel :

- on déduit le volume sanguin cardiopulmonaire moyen (CPBVmean), en faisant usage de l'équation

$$\texttt{CPBVmean = COmean*TTcp mean,}$$

dans laquelle COmean est le débit cardiaque (CO) moyen dans la phase de pression positive moyenne dans les voies respiratoires après stabilisation et TTcp mean est le temps de transit cardiopulmonaire moyen du sang déduit de l'enveloppe de la pression différentielle artérielle (5) et
- on déduit le temps moyen de transit cardiopulmonaire du sang (TTcp mean) en faisant usage des équations

$$\texttt{TTcp mean = t(D)-t(3-2)}$$

ou

$$\texttt{TTcp mean = t(F)-t(1-2),}$$

dans lesquelles t(3-2) est l'instant (15) de passage du niveau (10) le plus haut de PEEP ou de MPaw (PEEP 3) au niveau moyen de pression positive dans les voies respiratoires ou au niveau (17) intermédiaire de PEEP ou de MPaw (PEEP 2), t(1-2) est l'instant (12) de passage du niveau (11) le plus bas de PEEP ou de MPaw (PEEP 1) au niveau moyen de pression positive dans les voies aériennes ou au niveau (17) intermédiaire de PEEP ou de MPaw (PEEP 2), t(D) est l'instant (16) où l'enveloppe de la courbe de la pression différentielle artérielle (5) s'est adaptée à la PEEP intermédiaire ou au niveau de MPaw (17) et t(F) est l'instant (14) où l'enveloppe de la courbe de la pression différentielle artérielle (5) s'est adaptée à la PEEP intermédiaire ou au niveau de MPaw (17).

**25.** Programme d'ordinateur suivant l'une quelconque des revendications 22 à 24, ayant des instructions adaptées pour effectuer le stade dans lequel :

- on déduit le volume moyen du coeur gauche (LHVmean), en faisant usage de l'équation

$$LHVmean = COmean*TTlh,mean,$$

dans laquelle COmean est le débit cardiaque (CO) moyen dans la phase moyenne de pression positive dans les voies aériennes après stabilisation et TTlh,mean est le temps de transit moyen du sang déduit de l'enveloppe de la pression différentielle artérielle (5) et
- on déduit le temps de transit moyen du sang (TTlh,mean), en faisant usage de l'équation

$$TTlh,mean = t(1-2)-t(E),$$

dans laquelle t(E) est l'instant (13) où la courbe d'enveloppe de la pression différentielle artérielle (5) commence à s'élever.

26. Programme d'ordinateur suivant l'une quelconque des revendications 24 ou 25, ayant des instructions adaptées pour effectuer le stade dans lequel :

- on déduit la pente de la courbe de Starling pendant TriPAP pour le coeur total, en faisant usage du quotient de différence formé à partir de delta SV sur delta CPBV sur au moins deux niveaux de PEEP ou de MPaw et, pour le coeur gauche, en faisant usage du quotient de différence formé à partir de delta SV sur delta LHV sur au moins deux niveaux de PEEP ou de MPaw.

Fig.1

Fig.2

Fig.3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5769082 A
- US 20040249297 A
- EP 1155658 A
- US 6322514 B